# EUROPÄISCHE PATENTSCHRIFT

(11) **EP 3 801 710 B1**
(45) Veröffentlichungstag und Bekanntmachung des Hinweises auf die Patenterteilung: **28.09.2022**
(21) Anmeldenummer: 19735481.4
(22) Anmeldetag: 27.05.2019
(51) Int. Cl.: A61M 13/00

(54) **INSUFFLATIONSVORRICHTUNG**
INSUFFLATION DEVICE
DISPOSITIF D'INSUFFLATION

(30) Priorität: 25.05.2018 DE 102018004211
(43) Veröffentlichungstag der Anmeldung: 14.04.2021
(60) Teilanmeldung: 22191863.4
(73) Patentinhaber: W.O.M. World of Medicine GmbH, 10587 Berlin (DE)
(72) Erfinder: BISCHOF, Jan, 10719 Berlin (DE)
(74) Vertreter: Jungblut & Seuss
(86) Internationale Anmeldenummer: PCT/DE2019/000149
(87) Internationale Veröffentlichungsnummer: WO 2019/223826

(56) Entgegenhaltungen:
- WO-A1-2011/041387
- WO-A1-2015/043570
- DE-A1- 4 240 758
- DE-A1- 4 339 876
- DE-A1- 19 510 712
- US-A1- 2016 106 934
- US-B1- 6 299 592

## Beschreibung

Die vorliegende Erfindung betrifft einen Insufflator mit integrierter Rauchgasabsaugung. Durch eine neuartige Ventilregelung ist es möglich, den Gaszufluss über zwei Schläuche in die Körperhöhle einzuleiten.

### Hintergrund und Stand der Technik

Insufflatoren mit der Möglichkeit einer gleichzeitigen Rauchgasabsaugung sind aus dem Stand der Technik bekannt (siehe z. B. WO 2015/043570 A1). Dieser Insufflator weist einen Schlauch auf, durch den ein medizinisches Gas in eine Körperhöhle (z. B. ein Abdomen) eingeleitet wird. Das Gas erzeugt einen Überdruck, welches die Körperhöhle aufdehnt, damit ausreichend Platz für die visuelle Inspektion bzw. den therapeutischen Eingriff besteht. Über einen zweiten Schlauch wird das Gas aus dem Bauchraum wieder abgesaugt. Im Falle von therapeutischen Eingriffen mittels Elektrochirurgie oder Laser können gesundheitsschädliche Rauchgase entstehen, die durch den Insufflator über diesen zweiten Schlauch abgeführt und gefiltert werden.

US2016/106934 A1 beschreibt eine Insufflationvorrichtung mittels derer die abgesaugten Gase der Körperhöhle wieder zugeführt werden können (Zirkulation). Dabei können Rauchgase im Rahmen der Zirkulation gefiltert werden.

In der Praxis hat sich gezeigt, dass in bestimmten Situationen die im Stand der Technik bekannten Insufflatoren Nachteile aufweisen. Ein Beispiel hierfür sind größere Leckagen, die während der Operation auftreten. Hierfür werden Gasvolumenströme benötigt, die weit oberhalb der Maximalleistung bekannter Geräte von 20 bis 30 Liter/Minute liegen. Diese Limitierung kann aus Sicherheitsgründen nicht einfach durch Einstellung eines höheren Eingangsdruckes überwunden werden. Die Erhöhung des Eingangsdruckes würde zu einer Erhöhung des Patientenrisikos führen.

Die vorliegende Erfindung betrifft daher einen Insufflator für die minimal-invasive Chirurgie, enthaltend
a) Gasanschluss für eine Gasflasche oder Hausgas (1)
b) erste Druck- und Durchflussregeleinheit ausgestattet mit Proportionalventil und Drucksensor (3)
c)Zuführleitung (4, 6) mit erstem Filter (5) und Anschluss an einen ersten Trokar (7),
d) zweiter Trokar (8) mit Schlauch (9) und zweitem Filter (10), angeschlossen an eine Absaugvorrichtung mit regelbarer Absaugleistung (14),
e) elektronische Regelungseinheit, gekennzeichnet durch eine weitere Leitung (19, 17) im Insufflator, welche zwischen Gasversorgung (1) und der ersten Druck- und Durchflussregeleinheit (3) abzweigt und über eine zweite Druck- und Durchflussregeleinheit ausgestattet mit Proportionalventil und Drucksensor (18) zur Absaugleitung zwischen zweitem Filter (10) und Absaugvorrichtung (14) führt, so dass über Schlauch (9) und zweitem Trokar (8) eine zusätzliche Gaszufuhr zum Patienten ermöglicht wird, wobei optional zwischen zweitem Filter (10) und Absaugvorrichtung (14) ein Absperrventil (13) eingerichtet ist.

Im Gegensatz zu Insufflatoren aus dem Stand der Technik enthält der erfindungsgemäße Insufflator daher zwei Druck- und Durchflussregeleinheiten jeweils mit Proportionalventil und Drucksensor.

Im Normalfall wird der Insufflator so betrieben, wie es im Stand der Technik (WO 2015/043570 A1) beschrieben ist. Der Gasanschluss führt über die erste Druck- und Durchflussregeleinheit zur ersten Zuführleitung. Im Rahmen der Druck- und Durchflusseinheit ist ein Drucksensor eingerichtet, um den Druck in der Leitung zu überwachen. Ferner ist ein Filter zum Schutz des Patienten vorgesehen. Die erste Zuführleitung mündet in einen ersten Trokar, der die Körperhöhle mit Gas befüllen kann. Der Insufflator enthält weiterhin einen zweiten Schlauch, der im Normalfall als Absaugschlauch dient. Ein zweiter in die Körperhöhle eingeführter Trokar wird mittels dieses Absaugschlauches mit dem Insufflator verbunden. Auch der Absaugschlauch enthält einen Filter und führt zu einer Absaugpumpe. Die Absaugleistung der Absaugpumpe ist elektronisch regelbar. Die Regelungseinheit ist darauf eingerichtet, den Druck in der Körperhöhle soweit wie möglich konstant zu halten.

In einer optionalen Ausführungsform der Erfindung befindet sich keine Absaugpumpe im Insufflatorgehäuse. Stattdessen wird eine externe Pumpe verwendet, z.B. eine in Krankenhäusern übliche Wandabsaugung. In diesem Fall enthält das Insufflatorgehäuse ein Regelventil, welches den Gasstrom der Absaugung regelt. Es ist selbstverständlich möglich, dass der Insufflator beide Optionen enthält (Absaugpumpe und Anschluss an eine externe Pumpe).

Der maximale Volumenstrom eines derartigen Gerätes beträgt auf Grund verschiedener Begrenzungen typischerweise zwischen 20 und 30 lpm (Liter/Minute). Ein höherer Volumenstrom würde einen höheren Druck und/oder Trokare mit größerem Durchmesser erfordern. Beides ist aus Gründen der Sicherheit und Bedienbarkeit unerwünscht. In der Praxis treten allerdings Situationen auf, in denen der maximale Volumenstrom unzureichend ist, um den erwünschten Solldruck in der Körperhöhle aufrechter zu erhalten. In diesem Fall musste bislang die Operation pausiert und die Leckage geschlossen oder bei tieferem Druck in der Kavität weiteroperiert werden. Der erfindungsgemäße Insufflator weist für derartige Situationen eine zweite Druck- und Durchflussregeleinheit mit Proportionalventil auf. Mittels eines Stellventils ist es möglich, die Absaugpumpe vom Absaugschlauch zu trennen und stattdessen den Absaugschlauch als zweiten Zuführschlauch auszugestalten. Im Ergebnis kann somit über den zweiten Schlauch ebenfalls Gas zugeführt werden, sodass sich ein verdoppelter maximaler Gesamtvolumenstrom von bis zu 40-60 Liter/Minute erzeugen lässt.

Der erfindungsgemäße Insufflator kann aus Standardkomponenten (z.B. Pumpen, Ventile, Verbindungsleitungen, etc.) gebaut werden, die aus vielen Druckschriften bekannt sind und daher an dieser Stelle keiner weiteren Erläuterung bedürfen.

### Verbessertes Druckmessverfahren

Der erfindungsgemäße Insufflator ermöglicht auch eine verbesserte Druckregelung und -messung während der Insufflation. Bei den gängigen Insufflatoren wird die Druckmessung, die den Druck der Körperhöhle messen soll, durch einen Sensor vorgenommen, der am insufflatorseitigen Ende des Schlauches in der Druck- und Durchflussregeleinheit angeordnet ist. Um den Druck in der Körperhöhle messen zu können, wird die Insufflation für einige hundert Millisekunden unterbrochen. In dieser Zeit stellt sich ein Druckgleichgewicht zwischen der Körperhöhle und dem Schlauchinhalt ein, der dann gemessen werden kann. Damit kann der Druck in der Körperhöhle hinreichend genau bestimmt werden, ohne einen Drucksensor in der Körperhöhle einsetzen zu müssen. Nach Beendigung der Druckmessung wird die Insufflation fortgesetzt. Auf diese Weise kommt es zu einer pulsatilen Gaszufuhr bei welcher sich Messphasen mit Insufflationsphasen abwechseln. Dieser Wechsel ist für Körperhöhlen normaler Größen (z.B. Abdomen) unkritisch. Bei kleinen Körperhöhlen wie beispielsweise dem Rektum, führt diese Betriebsweise jedoch prinzipbedingt zu induzierten Druckschwankungen in der Körperhöhle.

Der erfindungsgemäße Insufflator erlaubt eine verbesserte Betriebsweise: Auch beim erfindungsgemäßen Insufflator wird die Gaszufuhr in der Zuführleitung für den Zeitraum der Druckmessung unterbrochen. Gleichzeitig mit der Unterbrechung wird die Gaszufuhr über die zweite Leitung geführt. Diese Art der Gaszuführung erlaubt eine ungestörte Messung des Druckes in der Kavität durch den ersten Drucksensor, während gleichzeitig der Gaszufluss kontinuierlich weiterbesteht.

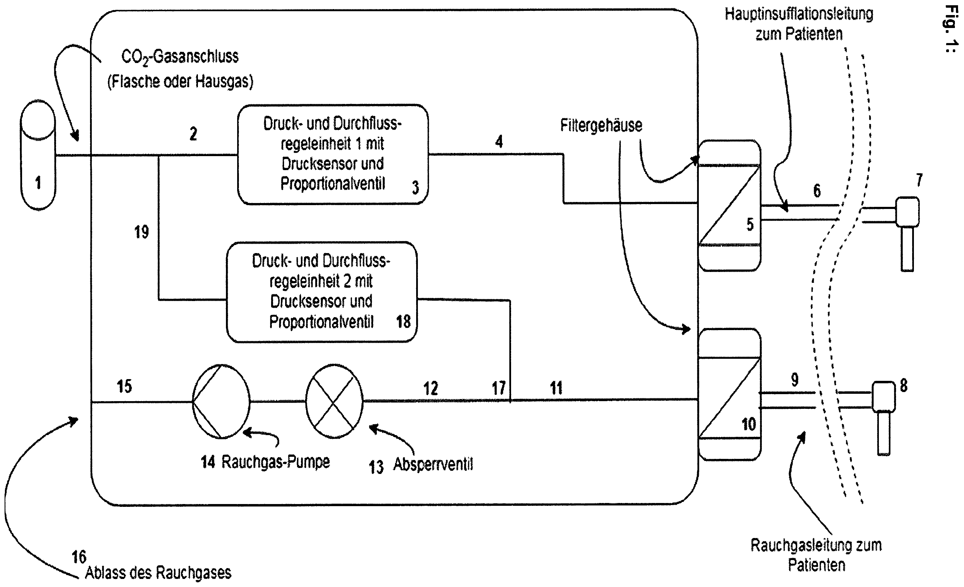

Die erfindungsgemäße Vorrichtung wird durch die Figur 1 illustriert. Zu sehen ist zunächst die Gasversorgung (1) (z. B. eine CO₂-Flasche). Die Gasversorgungsleitung (2) führt im Inneren des Insufflators zu einem ersten Druck- und Durchflussregeleinheit (3). In der Druck- und Durchflusseinheit befindet sich ein Proportionalventil sowie ein Drucksensor und Durchflusssensor. Die Leitung (4) führt zu einem Filter (5) am Gehäuseausgang. Mittels eines Schlauches (6) ist ein Trokar (7) angeschlossen. Dieser Trokar (7) führt den Gasstrom in die Kavität des Patienten. In die Kavität des Patienten ist ein zweiter Trokar (8) eingelassen. Durch diesen zweiten Trokar (8) findet im Regelfall eine Gasabsaugung statt. Der Trokar ist zu diesem Zweck mittels eines Schlauches (9) mit einem weiteren Filter (10) verbunden. Die Leitung (11) führt über ein optionales Absperrventil (13) zur Absaugpumpe (14) und von dort über eine Leitung (15) zu einem Ablass (16) für das abgesaugte Rauchgas. Zwischen Filter (10) und Absperrventil (13) ist eine zweite Druck- und Durchflussregeleinheit (18) mittels eines T-Stückes (17) positioniert. Die zweite Druck- und Durchflusseinheit ist ebenfalls über eine Leitung (19) mit dem Gasanschluss (1) verbunden.

Über diese zweite Leitung (9) kann Insufflationsgas dem zweiten Trokar (8) zugeführt werden. Das Absperrventil (13) muss geschlossen sein, wenn die Parallelinsufflation über den zweiten Trokar (8) erfolgen soll. Auf das Absperrventil kann dann verzichtet werden, wenn die Absaugpumpe einen hinreichenden Widerstand oder Trägheit aufweist und der Druck sich nicht über die Pumpe abbauen kann. Durch diese Parallelinsufflation über beide Trokare (7 und 8) kann ein praktisch doppelt so hoher Insufflationsvolumenstrom erzeugt werden, wie mit nur einer Leitung. Diese Parallelinsufflation erlaubt die Aufrechterhaltung eines ausreichenden Druckes im Patienten in besonderen Fällen, z. B. größeren Leckagen. Die elektronische Regeleinheit ist in der Figur 1 nicht dargestellt.

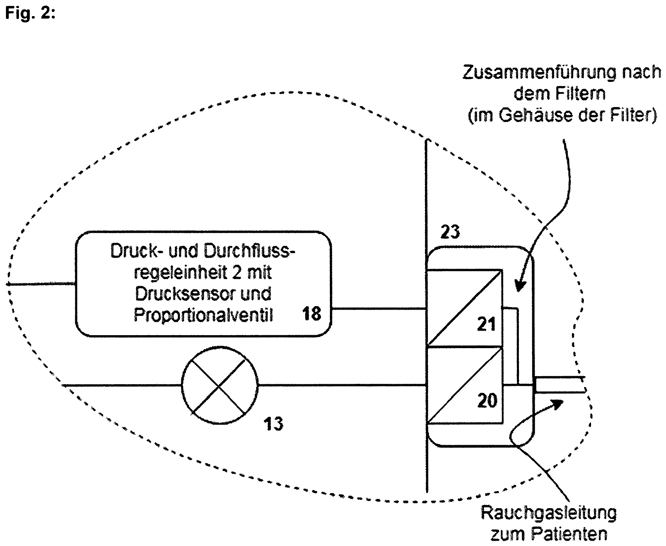

Im praktischen Betrieb wird der Filter (10) mit den Rauchgasemissionen kontaminiert. Um zu vermeiden, dass Partikel, und insbesondere Keime, bei Nutzung des zweiten Insufflationsweges vom Filter (10) über den Trokar (8) wieder in die Bauchhöhle eines Patienten zurückgelangen, besteht natürlich die Möglichkeit, die Leitung der zweiten Druck- und Durchflussregeleinheit (18) erst nach dem Filter (10) in die Leitung zum Trokar (8) münden zu lassen. Hierfür ist ebenfalls ein entsprechendes Filtergehäuse (23) zu konstruieren, welches gleichzeitig die Filterung der Rauchgase über einen Filter (20) ermöglicht, aber auch die Einmündung des Gasstromes von der zweiten Druck- und Durchflussregeleinheit (18) über einen Filter (21) zum Trokar (8) erlaubt. Eine derartige Lösung ist beispielsweise in der Figur 2 dargestellt.

Die erfindungsgemäße Insufflationseinheit erlaubt auch eine verbesserte Druckmessung im Regelbetrieb. Wie eingangs bereits geschildert, wird zum Zwecke der Druckmessung am Drucksensor das Ventil in der Druck- und Durchflusseinheit (3) für einige hundert Millisekunden geschlossen. Nach Druckausgleich zwischen der Kavität des Patienten und dem Leitungssystem wird der Druck über den Drucksensor festgestellt. Nach erfolgter Druckmessung wird das Proportionalventil in der Druck- und Durchflusseinheit (3) wieder geöffnet und die Insufflation fortgesetzt. Während der Druckmessung ist in den üblichen Insufflatoren keine weitere Insufflation möglich. Der erfindungsgemäße Insufflator ermöglicht eine kontinuierliche Insufflation durch folgendes Betriebsverfahren:
Zum Zweck der Druckmessung wird das Proportionalventil in der ersten Druck- und Durchflussregeleinheit (3) geschlossen. Gleichzeitig wird das Ventil in der zweiten Druck- und Durchflussregeleinheit (18) geöffnet, sodass die Insufflation für einige hundert Millisekunden mit gleicher Insufflationsleistung über den Trokar (8) durchgeführt wird. Sofern eine Rauchgasabsaugung stattfindet, kann für die Zeit der Druckmessung das Absperrventil (13) geschlossen werden. Nach erfolgter Druckmessung wird das Proportionalventil in der zweiten Druck- und Durchflussregeleinheit (18) wieder geschlossen und das Proportionalventil in der ersten Druck- und Durchflussregeleinheit (3) wieder geöffnet. Gegebenenfalls wird auch das Steuerungsventil (13) wieder geöffnet, sodass eine Rauchgasabsaugung möglich ist. Der Vorteil dieses Betriebsverfahrens ist, dass sich eine kontinuierliche Insufflation ergibt und damit der Druck in der Kavität somit deutlich weniger schwankt, als bei herkömmlichen Verfahren.

## Patentansprüche

1. Insufflator für die minimal-invasive Chirurgie, enthaltend
a) Gasanschluss für eine Gasflasche oder Hausgas (1)
b) erste Druck- und Durchflussregeleinheit ausgestattet mit Proportionalventil und Drucksensor (3)
c) Zuführleitung (4, 6) mit erstem Filter (5) und Anschluss an einen ersten Trokar (7),
d) zweiter Trokar (8) mit Schlauch (9) und zweitem Filter (10), angeschlossen an eine Absaugvorrichtung mit regelbarer Absaugleistung (14),
e) elektronische Regelungseinheit,
**gekennzeichnet durch** eine weitere Leitung (19, 17) im Insufflator, welche zwischen Gasversorgung (1) und der ersten Druck- und Durchflussregeleinheit (3) abzweigt und über eine zweite Druck- und Durchflussregeleinheit ausgestattet mit Proportionalventil und Drucksensor (18) zur Absaugleitung zwischen zweitem Filter (10) und Absaugvorrichtung (14) führt, so dass über Schlauch (9) und zweitem Trokar (8) eine zusätzliche Gaszufuhr zum Patienten ermöglicht wird, wobei optional zwischen zweitem Filter (10) und Absaugvorrichtung (14) ein Absperrventil (13) eingerichtet ist.

2. Insufflator gemäß Anspruch 1, **dadurch gekennzeichnet, dass** die Absaugvorrichtung mit regelbarer Absaugleistung (14) im Insufflatorgehäuse angeordnet ist.

3. Insufflator gemäß Anspruch 1, **dadurch gekennzeichnet, dass** die Absaugvorrichtung mit regelbarer Absaugleistung (14) als externe Pumpe ausgebildet ist, wobei das Insufflatorgehäuse ein Regelventil enthält, welches den Gasstrom der Absaugung regelt.

4. Insufflator gemäß Anspruch 1, **dadurch gekennzeichnet, dass** die Absaugvorrichtung mit regelbarer Absaugleistung (14) im Insufflatorgehäuse angeordnet ist und dass ein Anschluss an eine externe Pumpe eingerichtet ist.

## Claims

1. An insufflator for minimally invasive surgery, comprising
a) a gas port for a gas cylinder or domestic gas (1),
b) a first pressure and flow regulation unit (3) equipped with a proportional valve and a pressure sensor,
c) a supply line (4, 6) with a first filter (5) and a connection to a first trocar (7),
d) a second trocar (8) with a tube (9) and a second filter (10), connected to a suction device (14) with adjustable suction power,
e) an electronic regulation unit,
**characterized by** another line (19, 17) in the insufflator, which branches off between the gas supply (1) and the first pressure and flow regulation unit (3) and leads via a second pressure and flow regulation unit (18) equipped with a proportional valve and a pressure sensor, to the suction line between the second filter (10) and the suction device (14), so that via the tube (9) and the second trocar (8) an additional gas supply to the patient is enabled, optionally between the second filter (10) and the suction device (14) a shutoff valve (13) being arranged.

2. ) The insufflator according to claim 1, **characterized by that** the suction device (14) having an adjustable suction power is arranged in the insufflator housing.

3. ) The insufflator according to claim 1, **characterized by that** the suction device (14) having an adjustable suction power is configured as an external pump, the insufflator housing comprising a control valve, which regulates the gas flow of the suction device.

4. ) The insufflator according to claim 1, **characterized by that** the suction device (14) having an adjustable suction power is arranged in the insufflator housing, and that a connection to an external pump is provided.

## Revendications

1. Insufflateur pour la chirurgie mini-invasive, comprenant
a) un raccordement au gaz pour une bouteille de gaz ou au gaz à usage domestique (1),
b) une première unité de régulation de pression et de débit (3) équipée d'une vanne proportionnelle et d'un capteur de pression,
c) une conduite d'alimentation (4, 6) avec un premier filtre (5) et un raccordement à un premier trocar (7),
d) un deuxième trocar (8) avec un tuyau (9) et un deuxième filtre (10), raccordé à un dispositif d'aspiration (14) à puissance d'aspiration ajustable,
e) une unité de régulation électronique,
**caractérisé en** une conduite additionnelle (19, 17) dans l'insufflateur, qui est montée en dérivation entre l'alimentation de gaz (1) et la première unité de régulation de pression et de débit (3) et mène par l'intermédiaire d'une deuxième unité de régulation de pression et de débit (18) équipée d'une vanne proportionnelle et d'un capteur de pression à la conduite d'aspiration entre le deuxième filtre (10) et le dispositif d'aspiration (14), de façon que par l'intermédiaire du tuyau (9) et le deuxième trocar (8) une alimentation additionnelle de gaz au patient soit permise, optionnellement entre le deuxième filtre (10) et le dispositif d'aspiration (14) un clapet (13) étant arrangé.

2. ) Insufflateur selon la revendication 1, **caractérisé en ce que** le dispositif d'aspiration (14) à puissance d'aspiration ajustable est arrangé dans le boîtier de l'insufflateur.

3. ) Insufflateur selon la revendication 1, **caractérisé en ce que** le dispositif d'aspiration (14) à puissance d'aspiration ajustable est configuré comme une pompe externe, le boîtier de l'insufflateur comprenant une vanne de régulation, qui règle le débit du gaz du dispositif d'aspiration.

4. ) Insufflateur selon la revendication 1, **caractérisé en ce que** le dispositif d'aspiration (14) à puissance d'aspiration ajustable est arrangé dans le boîtier de l'insufflateur, et qu'un raccordement à une pompe externe est prévu.
